# EUROPEAN PATENT APPLICATION

(11) **EP 2 471 514 A1**
(43) Date of publication of application: **04.07.2012**
(21) Application number: 10197293.3
(22) Date of filing: 29.12.2010
(51) Int. Cl.: A61K 9/16, A61K 9/20, A61K 9/48, A61K 31/23

(54) **Controlled moisture content of cetyl myristate and/or cetyl palmitate granules or formulations**

(71) Applicant: Deva Holding Anonim Sirketi, Kucukcekmece/Istanbul (TR)
(72) Inventor: HAAS, Philipp Daniel, 34303, ISTANBUL (TR); FIRAT, Omer Faruk, 34303, ISTANBUL (TR); KANDEMIR, Levent, 34303, ISTANBUL (TR); KOC, Fikret, 34303, ISTANBUL (TR); SIVASLIGIL, Ramazan, 34303, ISTANBUL (TR)

(57) **Abstract**

This invention is directed to drying methods and determining of water content in granules or pharmaceutical/dietary supplement compositions or finished dosage forms of cetyl myristate or cetyl palmitate or combination of cetyl myristate and cetyl palmitate.

## Description

### Technical Field

This invention is related to drying methods and determining of water content in granules or pharmaceutical or dietary compositions or finished dosage forms of cetyl myristate or cetyl palmitate or combination of cetyl myristate and cetyl palmitate.

### Background Art

Cetyl palmitate is derived from the fatty acid, palmitic acid which occurs as the glycerol ester in many oils and fats such as palm oil or Chinese vegetable tallow. A synthetic method of preparation is to react palmitoyl chloride and cetyl alcohol in the presence of magnesium. See the Merck Index, 12th edition at page 336. Reference is also made to **US** US3169099 A (SOCONY MOBIL OIL CO INC) 02.09.1965 patent which discloses a biosynthetic method of producing cetyl palmitate.

US 4,113,881 A (DIEHL HARRY WELDON) 12.09.1978 discloses that the administration of an effective amount of cetyl myristoleate to a mammal is useful in inhibiting or relieving the symptoms of inflammatory rheumatoid arthritis in mammals.

US 5569676 A (DIEHL, HARRY W) 29.10.1996 US 5,569,676 discloses the use of cetyl myristoleate in the treatment of osteo-arthritis.

WO 01/85162 A (MERACOL CORP LTD ET.AL.) 15.11.2001 discloses the use of cetyl myristate and/or cetyl palmitate in the treatment of irritable bowel syndrome or disease. Patent embraces that cetyl myristate comprises 50-98 wt.% of the mixture, preferably, the myristate and palmitate are in a weight ratio of 95:5. The oral dosage unit is a capsule and contains 5-400 mg of the cetyl myristate or the mixture of the cetyl myristate and the cetyl palmitate. It also includes an excipient and/or diluent, preferably silicon dioxide, calcium phosphate and/or magnesium oxide. Preferably said mixture is in a capsule and it includes a pharmaceutically acceptable excipient and/or diluents. Preferably the dosage unit includes silicon dioxide, calcium phosphate and/or magnesium oxide. Liquid formulation, where an amount of liquid equivalent to at least 4 capsules is prescribed which is to be taken 3 times daily. That is 4200 mg of cetyl myristate or the mixture of cetyl myristate and cetyl palmitate.That mixture comprises by weight 95% cetyl myristate and 5% cetyl palmitate by weight In addition added excipients were present in the non gelatin two part capsule case.

WO 01/85163 A (MERACOL CORP LTD ET.AL.) 15.11.2001 discloses the use of cetyl myristate and/or cetyl palmitate in the treatment of eczema and/or psoriasis. Accordingly, capsule also includes a pharmaceutically acceptable excipient and/or diluent. These are silicon dioxide, calcium phosphate and/or magnesium oxide. The dosage unit can also be a wax-like solid or can be an orally consumable liquid composition (eg; made up with a general pharmacy type carrier such as methyl cellulose).

WO 2005/118070 A (MERACOL CORP LTD ET.AL.) 15.12.2005 discloses the treatment of multiple sclerosis with the use of cetyl myristate and/or cetyl palmitate.The cetyl myristate; or combination of cetyl myristate and cetyl palmitate is administered simultaneously, separately or sequentially.

WO 03/018731 A (MERACOL CORP LTD) 06.03.2003 defines the process prepares a mixture of cetyl myristate (50-98 wt.%) and cetyl palmitate, for use in the formulation of cosmetics and pharmaceuticals.

WO 03/045374 A (MERACOL CORP LTD ET.AL.) 05.06.2003 discloses the use of cetyl myristate and/or cetyl palmitate in a method of treatment and/or prophylaxis of a mammal for at least the symptoms of treating asthma, chronic obstructive pulmonary disease and/or other respiratory difficulties.

WO 03/026640 A (MERACOL CORP LTD ET.AL.) 03.04.2003 discloses the use of cetyl myristate and/or cetyl palmitate in the treatment of food allergies and/or food intolerances.

WO 01/85164 A (MERACOL CORP LTD ET.AL.) 15.11.2001 discloses the use of cetyl myristate and/or cetyl palmitate in the treatment of herpes.

### Summary of invention

This invention discloses granules or pharmaceutical and/or dietary supplement composition or finished dosage forms of cetyl myristate or cetyl palmitate or combination of cetyl palmitate and cetyl myristate having water content from 0.1 % to 5 % by weight or 0 % to 5 % by weight
wherein drying method is heating or vacuuming respectively.

### Technical Problem

If drying of granules or pharmaceutical compositions or pharmaceutical finished dosage forms of cetyl myristate or cetyl palmitate or combination of cetyl myristate and cetyl palmitate is fulfilled by heating it is faced that dried materials become adhesive or gel or quasi-gel forms thus tabletting or blistering or filling into capsules is very difficult or impossible.

### Solution to Problem

To solve the technical problem, it is invented that granules or pharmaceutical or dietary supplement compositions or finished dosage forms of cetyl myristate or cetyl palmitate or combination of cetyl myristate and cetyl palmitate are dried by heating till obtaining water content from 0.1% to 5 % by weight. If drying is carried out by vacuuming without heating, lack of water content is also accepted , thus in vacuuming water content is from 0 % to 5 % by weight. Water content of granules or pharmaceutical/dietary supplement compositions or finished dosage forms of cetyl myristate or cetyl palmitate or combination of cetyl myristate and cetyl palmitate from 0.1 % to 5 % (in heating) or 0 % to 5% (in vacuuming without heating) by weight provides eligible and elegant processing such as blistering, tabletting or filling into capsule. It is also invented that drying is executed by vacuuming without heating.

### Description of embodiments

In accordance with this invention cetyl myristate or cetyl palmitate or combination of cetyl myristate and cetyl palmitate is used as active pharmaceutical ingredients (API) in pharmaceutical or dietary supplement formulations.

In this invention the terms of "granule" or "pharmaceutical composition" or "pharmaceutical finished dosage form" encompass "dietary supplement granule" or "dietary supplement composition" or "dietary supplement finished dosage form" respectively.

Determining of water content is vital for drying in order to obtain suitable material of cetyl myristate or cetyl palmitate or combination of cetyl myristate and cetyl palmitate.

Powder flow, compaction,processability and such properties are influenced by the presence of moisture.

If drying is rendered by heating and water content exceeds beyond to 5 % by weight or if water content is reduced to under 0.1 % by weight, sticking or gelling problems are taken place.

When water content is reduced to under 0.1 % by weight and drying is fulfilled by heating, cetyl myristate or cetyl palmitate or combination of cetyl myristate and cetyl palmitate become adhesive or gel or quasi-gel forms thus blistering or tabletting or filling into capsules or such processing is very difficult or impossible. Its rationale is that completely removing of water content to zero needs more heating and more heating causes adhesive or gel or quasi-gel forms. Without heating, natural drying, namely leaving of material to room temperature or ambient temperature, cannot be efficient in industrial scale.

Where water content exceeds 5 % by weight, wet form of cetyl myristate or cetyl palmitate or combination of cetyl myristate and cetyl palmitate bedaubs to equipments thus blistering, tabletting or filling into capsules is very difficult or impossible.

According to this invention water content of granules or pharmaceutical compositions or pharmaceutical finished dosage forms of cetyl myristate or cetyl palmitate or combination of cetyl myristate and cetyl palmitate is not more than 5% by weight and is not less than 0.1 % by weight if drying is executed by heating.

Thus the water content contemplated by the present invention is from 0.1 % to 5 % after drying.

Preferably, water content is from 0.3% to about 4%; more preferably, from about 0.5% to about 3.5 %; and, most preferably, from about 0.6 % to about 2 % by weight.

In accordance with this invention materials are dried by a method selected from the group consisting of microwave drying, vacuum drying, hot air drying, infrared drying, drying oven and fluid bed.

In another aspect, this invention provides vacuum drying of granules or pharmaceutical compositions or pharmaceutical finished dosage forms of cetyl myristate or cetyl palmitate or combination of cetyl myristate and cetyl palmitate. Vacuum drying process also requires that at the end of drying water content is not more than 5% by weight. But no water content is acceptable in vacuum drying since it does not require heating thus there is no conversion to adhesive or gel forms.

Vacuum drying without heating is executed by use of different apparatuses. For instance drying may be carried out by use of Collette Ultima Pro 600 or Glatt WSG 200 thanks to their vacuum functions without heating.

According to this invention drying may separately be made or drying may be a part of granulation, blistering, tabletting or filling the capsule.

According to invention, to determine moisture content IR method is used through HR83 Halogen Moisture Analyzer (METTLER TOLEDO). Sample is held at 105 °C during 10 minutes and weight/weight method is used.

## Claims

1. Pharmaceutical or dietary supplement granules or pharmaceutical or dietary supplement compositions or pharmaceutical or dietary supplement finished dosage forms of cetyl myristate or cetyl palmitate or combination of cetyl myristate and cetyl palmitate **characterized in that** water content is from 0.1 % to 5 % by weight.

2. Water content, as claimed in claim 1, is preferably from 0.3% to 4% by weight

3. Water content, as claimed in claim 1, is more preferably from 0.5% to 3.5 % by weight

4. Water content, as claimed in claim 1, is most preferably from 0.6 % to 2 % by weight.

5. According to preceding claims, drying is carried out by heating.

6. Drying, as claimed as claim 5, is carried out by a method selected from the group consisting of microwave drying, vacuum drying, hot air drying, infrared drying, drying oven and fluid bed.

7. Pharmaceutical or dietary supplement granules or pharmaceutical or dietary supplement compositions or pharmaceutical or dietary supplement finished dosage forms of cetyl myristate or cetyl palmitate or combination of cetyl myristate and cetyl palmitate **characterized in that** water content is from 0% to 5 % by weight and drying is performed by vacuuming without heating.

8. According to preceding claims, water content is determined by using IR techniques.

9. According to claim 8 to determine moisture content HR83 Halogen Moisture Analyzer (Mettler Toledo) is used and sample is held at 105 °C during 10 minutes and weight/weight method is applied.
